(19) Europäisches Patentamt
European Patent Office
Office européen des brevets

(11) **EP 1 925 939 A1**

(12) **EUROPEAN PATENT APPLICATION**
published in accordance with Art. 153(4) EPC

(43) Date of publication:
**28.05.2008 Bulletin 2008/22**

(51) Int Cl.:
***G01N 33/545*** (2006.01)

(21) Application number: **05767225.5**

(22) Date of filing: **28.07.2005**

(86) International application number:
**PCT/JP2005/013807**

(87) International publication number:
**WO 2007/013157 (01.02.2007 Gazette 2007/05)**

(84) Designated Contracting States:
**AT BE BG CH CY CZ DE DK EE ES FI FR GB GR HU IE IS IT LI LT LU LV MC NL PL PT RO SE SI SK TR**

(71) Applicant: **KABUSHIKI KAISHA YAKULT HONSHA Minato-ku, Tokyo 105-8660 (JP)**

(72) Inventors:
- **HASHIMOTO, Toshikazu, Japan Sewage Works Agency Tokyo 1070052 (JP)**
- **MISHINA, Fumio, c/o Japan Sewage Works Agency Tokyo 1070052 (JP)**
- **OKUMURA, Takekazu, Kabushiki Kaisha Yakult Honsha Tokyo 1058660 (JP)**
- **NAGAI, Fumiko, c/o Kabushiki Kaisha Yakult Honsha, Tokyo 1058660 (JP)**
- **YAMAMOTO, Shuta, c/o Kabushiki Kaisha Yakult Honsha Tokyo 1058660 (JP)**
- **SAWADA, Haruji, c/o Kabushiki Kaisha Yakult Honsha, Tokyo 1058660 (JP)**

(74) Representative: **Warcoin, Jacques et al Cabinet Régimbeau 20, rue de Chazelles 75847 Paris Cedex 17 (FR)**

(54) **ANTIBODY-SENSITIZED LATEX**

(57) Antibody-sensitized latex for use in determination of a bacterium belonging to the genus *Nitrospira,* the latex comprising latex particles each having a specific gravity of about 1.5 g/mL and an average particle diameter in the range of 1.0-1.5 μm and an antibody adsorbed thereon. The antibody is specific to a bacterium belonging to the genus *Nitrospira* having an activity of nitrite oxidization. The latex is mixed with a sample, allowed to stand for a predetermined period of time, and observed on the presence or absence of agglutination of the latex particles in the mixture. This latex permits detection and determination of a bacterium belonging to the genus *Nitrospira* which is found in biological sewage treatment systems, such as streams and activated sludge, as well as in water of rivers, lakes and seashore, and in soil habitats, in a simple and easy manner.

Fig. 1

## Description

### Technical Field

The present invention relates to an antibody-sensitized latex to detect a bacterium belonging to the genus *Nitrospira* which is found in biological sewage treatment systems, such as streams and activated sludge, as well as in water of rivers, lakes and seashore, and in soil habitats. More particularly, the present invention relates to the antibody-sensitized latex which allows for accurate and rapid detection of a bacterium belonging to the genus *Nitrospira* unlike the conventional method that needs skillful work and long time. The present invention relates also to a method for determination of a bacterium belonging to the genus *Nitrospira* by using said antibody-sensitized latex and a method of applying said determination to process control of biological sewage treatment.

### Background Art

The human living environment involves waste water and raw garbage which contain organic matter or nitrogen compounds. They pose a serious environmental problem if they are discharged as such into rivers, lakes, and underground water. One way of addressing this problem is by biological purification through nitrification and denitrification that employs activated sludge in their disposal. This process is generally used in many sewage treatment facilities. One step of this process is nitrification in which nitrifying bacteria living in activated sludge play an important role.

Since nitrification limits the rate of biological sewage treatment process, it is essential to quantify microorganisms involved in nitrification (such as nitrifying bacteria, ammonia oxidizing bacteria, and nitrite oxidizing bacteria) for improvement in process stability and efficiency.

Being mostly autotrophic, bacteria involved in nitrification have a smaller specific growth rate than heterotrophic ones to remove organic matter, and they are subject to environmental conditions such as pH and water temperature. So, it is extremely important to keep high the density of nitrifying bacteria in the bioreactor for nitrification.

The operation and maintenance of the reactor are currently accomplished according to the results of quantitative analysis of input to and output from sewage treatment facilities, but information about microflora is hardly used for them.

Therefore, determination of nitrite oxidizing bacteria is necessary for biological sewage treatment systems and mud/sludge habitats in activated sludge tanks or reactors of sewage treatment facilities. The conventional method of indirect determination consists of cultivation for one to two months after sampling and measurement of the amount of nitrite consumed in that period (Japanese Article: Ryusuke Kimura et al., "Methods for Experiments on Soil Microorganisms", Society for the Research of Soil Microorganisms, Yokendo Co., Ltd., (1992) pp.207-214).

Attempts have been made to use the foregoing method for determination to control nitrifying bacteria in activated sludge or soil for the biological sewage treatment. Unfortunately, the foregoing method needs skillful work for measuring the nitrifying bacteria and needs a considerably long period of time, and hence it is not suitable for immediate daily control of the nitrifying bacteria from the measuring result.

To cope with this situation, there has been developed a method for detection of a bacterium belonging to the genus *Nitrosomonas* (ammonia oxidizing bacterium) and a bacterium belonging to the genus *Nitrobacter* (nitrite oxidizing bacterium), which uses an antibody capable of specifically recognizing these bacteria. The use of this method has been proposed for rapid and simple detection of the ammonia oxidizing bacterium and the nitrite oxidizing bacterium (JP-5322896-A). Another method proposed so far for detection in a simpler way employs an antibody-sensitized latex composed of latex particles and said antibody adsorbed thereon (JP-8029426-A).

Incidentally, knowledge about distribution and population of nitrifying bacteria are being gradually renewed since the molecular biological techniques were introduced into the analysis of water environment from 1990. Among new knowledge about nitrite oxidizing bacteria is the finding of a bacterium of the genus *Nitrospira.* Much has been reported about nitrification process in which this bacterium dominates in water being treated. Now, it is an important subject to accurately determine (count) not only bacteria of the genera *Nitromonas* and *Nitrobacter* but also bacteria of the genus *Nitrospira* in order to correctly know how efficiently the biological treatment process removes nitrogen.

Unfortunately, the foregoing conventional method for determination does not produce adequate results because other bacteria than mentioned above dominate during cultivation with sludge sampled from biological purification facilities. Another problem is unavailability of any antibody that specifically recognizes the bacterium belonging to the genus *Nitrospira,* which is due to the fact that no method was available for isolation of the bacterium belonging to the genus *Nitrospira.* Thus, the bacterium belonging to the genus *Nitrospira* presents difficulties in detection and determination by the conventional method.

### Disclosure of the Invention

With the foregoing in mind, the present inventors carried out a series of researches to develop a method for rapid and simple determination of a bacterium belonging to the genus *Nitrospira* which is found in biological sewage treatment systems, such as streams and activated sludge, as well as in water of rivers, lakes and seashore, and in soil habitats. As the result, the present inventors discovered the fact that the objective bacterium can be detected and determined in a simple and rapid manner by using an antibody-sensitized latex which comprises a liquid medium and latex particles suspending therein and carrying an antibody adsorbed thereon, said antibody being specific to the bacterium belonging to the genus *Nitrospira.* This finding led to the present invention.

Thus it is an object of the present invention to provide a new antibody-sensitized latex for rapid and simple detection of a bacterium belonging to the genus *Nitrospira* which is found in biological sewage treatment systems, such as streams and activated sludge, as well as in water of rivers, lakes and seashore, and in soil habitats. It is another object of the present invention to provide an immunoassay for rapid and simple determination of a bacterium belonging to the genus *Nitrospira* which is found in biological sewage treatment systems, such as streams and activated sludge, as well as in water of rivers, lakes and seashore, and in soil habitats, said immunoassay employing said antibody-sensitized latex. It is further object of the present invention to provide a method for controlling the daily operation of the biological sewage treatment process with aerobic bacteria in accordance with a cell count of nitrifying bacteria such as a bacterium belonging to the genus *Nitrospira.*

According to the present invention, the antibody-sensitized latex for detecting a bacterium belonging to the genus *Nitrospira* comprises a liquid medium and latex particles suspending therein. Each of the latex particles has a specific gravity of about 1.5 g/mL and an average particle diameter in the range of 1.0-1.5 $\mu$m. Each of the latex particles also carries an antibody adsorbed thereon, the antibody being specific to a bacterium belonging to the genus *Nitrospira* having an activity of nitrite oxidization. The antibody-sensitized latex allows for detection of the bacterium belonging to the genus *Nitrospira* in a simple and rapid manner without requiring skillful work and long time.

According to the present invention, the antibody-sensitized latex for determination of a bacterium belonging to the genus *Nitrospira* is suitably applied to determination of said bacterium by immunoassay. The immunoassay involves reactions between a sample and an antibody specific to the bacterium belonging to the genus *Nitrospira,* so that it permits determination and the counting of said bacterium. Samples for the immunoassay are those taken from biological sewage treatment systems, such as streams and activated sludge, as well as from water of rivers, lakes and seashore, and from soil habitats. The samples may be used as such or after dilution. Thus, the present invention also provides an immunoassay for detection and determination of a bacterium belonging to the genus *Nitrospira* in a sample with the help of an antibody specific to said bacterium.

The antibody-sensitized latex of the present invention is applied to the immunoassay which includes not only immune-agglutination, such as latex agglutination and reverse passive latex (erythrocyte) agglutination, but also immune-chromatography, immune-fluorescence, enzyme immunoassay and radioimmunoassay. Of these methods, immune-agglutination and immune-chromatography are desirable from the standpoint of simplicity and rapidity. Of the immune-agglutination methods, reverse passive latex (erythrocyte) agglutination is particularly suitable, which uses indirect agglutination reaction by erythrocytes or latex particles bound with antigen or antibody.

The insoluble carriers that can be used for immune-agglutination according to the present invention include red blood cells, particles of inorganic compound, such as colloidal gold particles, and particles of organic polymer, such as latex particles. Latex particles are preferable from the standpoint of stability after sensitization with an antigen or antibody.

According to the present invention, a bacterium belonging to the genus *Nitrospira* is applied to biological sewage treatment process. The bacterium belongs to the genus of nitrite oxidizing bacteria, which is a microorganism that converts nitrite into nitrate through oxidization. At present, 43 strains of 16S-rRNA base sequences of bacteria belonging to the genus *Nitrospira* have been registered at DDBJ (DNA Data Bank of Japan). Phylogenetic study based on these base sequences suggests that the genus contains four groups.

Only two strains, however, are known which have been identified by pure culture. They are *Nitrospira moscoviensis* isolated from fresh water and *Nitrospira marina* ATCC43039 isolated from deep sea. No strains are known which have been isolated from activated sludge, soil, and sludge in sewage treatment reactors or biological purification facilities. In Examples mentioned later, *Nitrospira moscoviensis* is used as a bacterium belonging to the genus *Nitrospira* for preparation of antibody. When any other strain than the foregoing two strains becomes available by pure culture in the future, it may be used for preparation of antibody as a matter of course.

The antibody specific to a bacterium belonging to the genus *Nitrospira* is obtained from an animal, such as rabbit, mouse, rat, fowl, sheep or horse, which has been immunized with said bacterium as the antigen. It may also be obtained from antiserum prepared by the above-mentioned known method (JP-5322896-A) which is followed by purification by affinity chromatography with protein G.

According to a preferred embodiment of the present invention, detection of a bacterium belonging to the genus *Nitrospira* by means of the antibody-sensitized latex mentioned above is accomplished by the steps of mixing a sample with the antibody-sensitized latex, allowing the resulting mixture to stand for a predetermined period of time, and observing the presence or absence of agglutination of latex particles in the mixture.

The sample should preferably be collected from the liquid being treated in a vessel or tank for swage treatment with aerobic bacteria, so that the bacterium belonging to the genus *Nitrospira* can be detected in the sample if they exist.

The following is a detailed explanation of a preferred embodiment of the present invention which is intended for detection of a bacterium belonging to the genus *Nitrospira* by immune-agglutination. The latex particles used for detection should be high-density ones having a specific gravity not lower than 1. 0 g/mL, preferably about 1. 5 g/mL, particularly in the range of 1.2-1.8 g/mL. They should be ones having an average particle diameter in the range of about 0.1-4.0 $\mu$m, preferably 1.0-1.5 $\mu$m, more preferably about 1.3 $\mu$m. Moreover, it is preferable that the latex particles are colored. Latex particles meeting these requirements are commercially available as, for example, "Bacto 0.81" (Trademark) from Difco Laboratories, having an average particle diameter of 0.81 $\mu$m and a specific gravity of 1.0 g/mL; "IMMUTEX-H Series" (Trademark) from JSR Corp., having an average particle diameter of 0.94-4.9 $\mu$m and a specific gravity of 1.5 g/mL, including colored ones; and "Polybeads" (Trademark) #15706 and #15709 from Polysciences, Inc. The latex particles used for the present invention are not restricted to the commercial ones cited above but any equivalent ones can be used.

There are several methods that can be applied for preparing the antibody specific to a bacterium belonging to the genus *Nitrospira.* One of them is as follows. First, the bacterium is cultivated in a liquid medium of inorganic salt with nitrite being a substrate for multiplication. The cultivated bacterium is sterilized (optionally) and collected for use as an antigen. This antigen is injected into the vein of a rabbit's ear. After an increase of antibody titer has been confirmed, whole blood is collected. The collected blood undergoes centrifugation and inactivation at 56°C to give antiserum. Finally, the antiserum is purified by affinity chromatography bound with protein G. In this way there is obtained the desired antibody.

The thus obtained specific antibody is adsorbed to latex particles as a carrier. The latex particles for this purpose or the sensitized latex may be prepared by any method such as the one disclosed in the aforementioned patent document (JP-8029426-A).

Particles carrying the specific antibody, which are used for immune-agglutination, may be prepared by physical adsorption method or chemical binding method, both of which are satisfactory for the present invention. In the case where latex particles are used for agglutination, physical adsorption makes latex particles sufficiently sensitized with the antigen or antibody.

Preferably, the latex particles are sensitized with the antigen or antibody by a process which is carried out in a buffer solution. That is, a suspension of latex particles, which is diluted with a buffer solution to an adequate concentration, is mixed with a solution of the antigen or antibody. After standing for a while, the latex particles are washed to give the sensitized latex particles as desired. The buffer solution used for dilution is one which has adequate ionic strength and pH value harmless to reactions between an antigen or antibody supported on the particles and an antigen or antibody to be determined. Examples of such buffer solutions include TBS, PBS, GBS, phosphate buffer solution, and borate buffer solution. Preferable among them are GBS, phosphate buffer solution, and borate buffer solution, which are less liable to cause agglutination or auto-agglutination of sensitized latex particles. The buffer solution should have pH 5-10, preferably pH 6-9.

The latex particles ready for sensitization should have a concentration of 0.01-0.5% (w/v), preferably 0.05-0.25% (w/v). The antibody solution ready for sensitization should have a concentration of 1.0-1,000 $\mu$g/mL in terms of antibody protein. Concentrations outside the foregoing range lead to a decrease in sensitivity and auto-agglutination of sensitized latex particles, which obscures judgment about positive and negative. In addition, reactions for sensitization should be carried out at 0-60°C, particularly room temperature to about 40°C. An adequate reaction temperature is necessary for highly sensitive latex particles.

It is preferable that the resulting sensitized latex is used for detection and determination of a bacterium belonging to the genus *Nitrospira* by the reverse passive latex agglutination method. This method is intended to detect or determine an object substance in a sample by mixing the sample with a solution (reagent) containing particles capable of immune-agglutination which are a high-density labeling substance (such as latex particles) carrying a substance (such as antibody) bound thereto which binds specifically to the object substance. To be concrete, the method comprises preparing a plurality of test samples which are different in degree of dilution of a sample to be determined, mixing each of the test samples with a reagent containing the antibody-sensitized latex to obtain a plurality of mixtures, and finding a degree of dilution of the sample that agglutination of the latex is observed among the mixtures. On the other hand, the same procedure as mentioned above is performed on the reference samples each of which is assigned to a known cell count of the bacterium. Thus, the cell count value of the bacterium in the sample is calculated from the degree of dilution and the known cell count of the bacterium.

According to a preferred embodiment of the present invention, a bacterium belonging to the genus *Nitrospira* is determined with the antibody-sensitized latex by the method which comprises the steps of: preparing a plurality of test samples which are different in degree of dilution of a sample to be determined; mixing each of the test samples with the antibody-sensitized latex to obtain a plurality of mixtures; allowing the mixtures to stand for a predetermined period of time; observing the presence or absence of agglutination of latex particles in each of the mixtures; comparing the result of observation of the mixtures with agglutination with the result of a reference test in which a procedure according to the foregoing steps are followed for a plurality of reference samples each of which is assigned to a known cell count of the bacterium belonging to the genus *Nitrospira;* and identifying the cell count of the bacterium belonging to the genus *Nitrospira* in the test samples on the basis of comparison of the result of observation of the mixtures with the result of the reference test which correspond to agglutination that observed in the mixtures.

Observation may be accomplished by using a microtiter plate having a number of wells with a round concave bottom. The wells of a first group receive a dispensed amount of one of the mixtures each containing the antibody-sensitized latex and one of the test samples diluted with a buffer solution in a degree of dilution which is different from each other, respectively. The wells of a second group receive a dispensed amount of one of mixtures each containing the antibody-sensitized latex and one of the reference samples each of which is assigned to a known cell count of the bacterium. The dispensed amount of the mixtures is the same for all wells. After standing at room temperature for 4-15 hours, the mixtures in the well are examined with the naked eye or a magnifier (x10) for agglutination of latex particles. The result of observation indicates the cell count of the bacterium. What is observed in a well which has received a buffer solution alone is regarded as negative, and this is compared with agglutination in other wells. The cell count of the bacterium contained in the sample is calculated from the degree of dilution of the test samples and the known cell count of the bacterium in the reference samples.

The foregoing procedure for detection and determination may be replaced by any other method, such as microscopic latex method, immune-chromatography, and immune-agglutination. The first one employs a slide glass in place of microtiter plate on which a sample is mixed with the antibody-sensitized latex particles, so that agglutination is observed under an optical microscope.

Incidentally, it has been known that latex particles are used as a carrier on which the antibody is adsorbed. However, nothing has been reported so far about the practical use of latex particles for detection and determination of a bacterium belonging to the genus *Nitrospira.* The effectiveness of latex particles was confirmed for the first time by the present inventors. In other words, the present inventors found that the antibody specific to a bacterium belonging to the genus *Nitrospira* is useful for selective detection and rapid determination of specific microorganisms capable of nitrite oxidization which is found in biological sewage treatment systems, such as streams and activated sludge, as well as in water of rivers, lakes and seashore, and in soil habitats, and that the result of such determination can be used to control the biological sewage treatment process.

According to another preferred embodiment of the present invention, the method for determining a bacterium belonging to the genus *Nitrospira* by using the antibody-sensitized latex further comprises the steps of measuring the cell count of a bacterium belonging to the genus *Nitrospira* which is found in the liquid being treated in a reactor for treatment with aerobic bacteria in order to control the operation of the reactor, and controlling at least one condition for operation selected from the group consisting of the residence time of solids in the reactor, the amount of dissolved oxygen in the reactor, and the inflow into the reactor, based on the measurement of the cell count of the bacterium, so as to maintain the cell count of the bacterium as a cell count necessary for the liquid in the reactor to keep the intended ratio of nitrification during treatment. Preferably, the cell count of the bacterium belonging to the genus *Nitrospira,* which is necessary for the liquid in the reactor to keep the intended ratio of nitrification, is maintained at $10^7$ cells/mL or more.

The foregoing steps may be modified such that the measurement of the cell count is performed on not only bacterium belonging to the genus *Nitrospira* but also bacteria belonging to the genera Nitrosomonas and Nitrobacter, all of which are found in the liquid being treated in a reactor for treatment with aerobic microorganisms, and the reactor is operated by controlling at least one condition for operation selected from the residence time of solids in the reactor, the amount of dissolved oxygen in the reactor, and the inflow into the reactor, based on the measurement of the cell count of the bacteria, so that the liquid in the reactor contains the bacteria as many as necessary to keep the intended ratio of nitrification during treatment.

The procedure mentioned above makes it possible to adequately control daily operation of facilities for treatment with aerobic bacteria by using as an index the cell count of major nitrifying bacteria including the bacterium belonging to the genus *Nitrospira* and others.

Incidentally, the term "treatment with aerobic bacteria" means a sewage treatment process of decomposing and removing by oxidization organic matter, ammonium nitrogen, odors, and iron with the help of a variety of aerobic bacteria. Treatment with aerobic bacteria falls under two broad classes. The first one is oxidization and decomposition of organic matter in an aerated liquid with bacteria floating in the form of flock. The second one is oxidization and decomposition of organic matter by contact of waste water with a microbial film composed of a support and aerobic microorganisms attached and proliferated thereon. The former is exemplified by the activated sludge process, and the latter is commonly called the biofilm process.

The activated sludge process mentioned herein includes the standard activated sludge process, oxygen activated sludge process, extended aeration process, oxidation ditch process, and batch activated sludge process. It also includes the circulating nitrification-denitrification process, nitrification-induced denitrification process, anaerobic-Anoxic-Oxic process, and anaerobic-aerobic activated sludge process. The biofilm process is now often called the immobilized process because it employs microorganisms immobilized on a support or carrier or contact material. The support may be either immersed (for fixed bed) or moved (for fluidized bed) in the reactor.

In the case where the microbial film is used for removal of nitrogen compounds and phosphorus from concentrated waste water, treatment with anaerobic bacteria is often combined with treatment with aerobic bacteria. Needless to say, the method for determination of the bacterium according to the present invention can be applied to the biological sewage treatment process which includes treatment with anaerobic bacteria.

As mentioned above, the present invention provides a novel antibody-sensitized latex for determination, which contains latex particles and an antibody adsorbed thereon which is specific to a bacterium belonging to the genus *Nitrospira.* This latex permits detection and determination of a bacterium belonging to the genus *Nitrospira* in a simple and rapid manner unlike the conventional technology that needs skillful work and long time. According to the present invention, it is possible to detect and identify a bacterium belonging to the genus *Nitrospira* which is found in biological sewage treatment systems, such as streams and activated sludge, as well as in water of rivers, lakes and seashore, and in soil habitats, owing to reaction with an antibody specific to a bacterium belonging to the genus *Nitrospira.* Moreover, according to the present invention, it is possible to determine the cell count of a bacterium belonging to the genus *Nitrospira* in a simple and rapid way. In addition, according to the present invention it is possible to control the conditions of operation of the treating tank by using the thus determined cell count of the bacterium as an index. This facilitates operation and control of sewage treatment facilities with aerobic bacteria.

**Best Mode for Carrying out the Invention**

The features and advantages of the present invention will be described in more detail with reference to some examples as shown below. Naturally, the technical scope of the present invention is not limited to these examples.

**Example 1: Preparation of bacteria for use as antigen**

*Nitrospira moscoviensis* was cultivated in a medium having the composition shown in Table 1 below. This strain was received from Dr. Eva Spieck of Universität Hamburg. (See Ehrich S., D. Behrens, E. Lebedeva, W. Ludwig, and E. Bock "A new obligately chemolitoautotrophic, nitrite-oxidizing bacterium, Nitrospira moscoviensis sp. nov. and its phylogenetic relationship" Arch. microbiol. 164 (1995), pp. 16-23.) Cultivation was carried out in three stages (seed culture in 100-mL scale, intermediate culture in 1-L scale, and final culture in 4-L scale). The culture temperature was 37°C and the culture period was 7 to 10 days in all stages.

The final culture was suspended when proliferation of the bacterium has reached a maximum at a guess. The culture medium at 4.0°C was centrifuged at 24,000 × g for 10 minutes to recover the bacterium. The bacterium was given 30 mL of buffer solution (PBS) and dispersed by pipetting. The resulting solution was thoroughly stirred with the equal amount of 0.6% formalin. The resulting solution was allowed to stand at 37°C for 1 hour, and then fixed at 4.0°C until the bacterium died. The fixed bacterium was recovered by centrifugation at 24,000 × g for 10 minutes, washed with PBS and then centrifuged again at 24,000 × g for 10 minutes. The thus recovered bacterium was used as an antigen. This antigen was diluted with PBS so that the resulting solution has an absorbance of 0.5 at a wavelength of 660 nm. The thus obtained antigen was used for the test.

Table 1

| Composition of medium (1,000 mL) for *Nitrospira moscoviensis,* strain DMS 10035 | | | |
| --- | --- | --- | --- |
| $NaNO_2$ | 0.2 g | $MnSo_4 \cdot H2O$ | 33.8 μg |
| $CaCO_3$ | 0.007 g | $H_3BO_3$ | 49.4 μg |
| NaCl | 0.5 g | $ZnSO_4 \cdot 7H_2O$ | 43.1 μg |
| $MgCl_2 \cdot 7H_2O$ | 0.05 g | $(NH_4)_6Mo_7O_{24} \cdot 4H2O$ | 37.1 μg |
| $KH_2PO_4$ | 0.15 g | $CuSO_4 \cdot 5H_2O$ | 25.0 μg |
| | | $FeSO_4 \cdot 7H_2O$ | 973.0 μg |
| | | | pH = 8.6 |

The purity of the antigen was confirmed by using DNA probes NSR1156 and Ntspa0662 specific to *Nitrospira.* In other words, the probes were used for hybridization to see that the bacteria suspension does not contain any other strain than *Nitrospira moscoviensis,* strain DMS 10035.

**Example 2: Preparation of polyclonal antibody**

Polyclonal antibody was prepared by using two rabbits KBL:Jw (Japanese white species). The antigen was administered at a dose of 0.5 mL each time by injection into the ear vein at intervals of 7 to 10 days. The serum of the rabbits being immunized was periodically sampled to see the increase of antibody titer for *Nitrospira moscoviensis,* strain DMS 10035, by ELISA (enzyme-linked immunosorbent assay).

The ELISA was carried out as follows. The antigen was diluted with a carbonate-bicarbonate buffer solution so that the resulting solution had an absorbance of $A_{660nm}$ = 0.05. The antigen solution was dispensed to 96 wells on a microtiter plate, 100 $\mu$L per well. The dispensed solution was allowed to stand for adsorption at 4.0°C for 16 hours. Each well was washed with PBS (-) containing 0.05% Triton-X100. Each well was given 120 $\mu$L of blocking solution (carbonate-bicarbonate buffer solution containing 1.0% BSA), followed by reaction at 37°C for 1 hour.

After washing, each well was given 90 $\mu$L of the serum which had been diluted serially with PBS(-) containing 0.05% Triton-X100 and 1.0% BSA, followed by reaction at 37°C for 1.5 hours. After washing again, each well was given 100 $\mu$L of peroxidase-labeled secondary antibody (anti-rabbit immunoglobulin G (IgG) -sheep IgG), followed by reaction at 37°C for 1.5 hours in the dark.

After the reaction was completed, each washed well was given 100 $\mu$L of citrate buffer solution with O-phenylenediamine containing hydrogen peroxide, followed by standing at 37°C for 10 minutes in the dark to induce color development. Each well was given 50 $\mu$L of 2.5M sulfuric acid to suspend the reaction, followed by measurement of absorbance ($A_{492nm}$) at a wavelength of 492 nm. Incidentally, the antibody titer was expressed in terms of the reciprocal of the dilution ratio at which the absorbance ($A_{492nm}$) of serum is 0.5. In the case where the antibody titer does not increase, whole blood is collected from the heart and the collected blood is inactivated at 56°C to prepare antiserum against the bacterium belonging to the genus *Nitrospira.*

**Example 3: Preparation of immunoglobulin G (IgG)**

The antiserum obtained in Example 2 underwent affinity chromatography with protein G (Mab Trap G Kit, from Amersham Pharmacia) for fractionation of IgG. The resulting purified IgG was used as anti-*Nitrospira* antibody in the following experiments.

**Example 4: Experiment on cross-reactivity**

The anti-*Nitrospira* antibody obtained in Example 3 was examined by ELISA for cross-reactivity with each bacteria shown in Table 2. The cross-reactivity is expressed in percentage of the relative antibody titer of the antibody in question for each bacterium, with the antibody titer of the anti-*Nitrospira* antibody for *Nitrospira moscoviensis,* strain DMS 10035, being 100%. The antibody titer is a reciprocal of the dilution at which the color reaction due to ELISA gives an absorbance $OD_{492}$ =0.5. Incidentally, it has often been reported that Gram-negative bacilli (such as *Pseudomonas, Alcaligenes* and *Flavobacterium)* dominate in activated sludge collected from water purification facilities.

Table 2
Strains used for examination of cross-reactivity with anti-*Nitrospira* antibody

| No. | Name of Strain | Culture Collection No. |
|---|---|---|
| 1 | *Agrobacterium radiobacter* | IFO 13532T |
| 2 | *Agrobacterium tumefaciens* | IFO 12307 |
| 3 | *Alcaligenes denitrificans* | IFO 15125T |
| 4 | *Alcaligenes faecalis* | IFO 13111T |
| 5 | *Bacillus licheniformis* | IFO 12200T |
| 6 | *Bacillus subtilis* | IFO 13719T |
| 7 | *Enterobacter* sp. | isolate |
| 8 | *Flavobacterium ferrugineum* | IFO 14992T |
| 9 | *Flavobacterium* sp. | IFO 15128 |
| 10 | *Flavobacterium* sp. | IFO 15147 |
| 11 | *Flavobacterium* sp. | isolate |
| 12 | *Nocardia amarae* | isolate |

(continued)

Strains used for examination of cross-reactivity with anti-*Nitrospira* antibody

| No. | Name of Strain | Culture Collection No. |
|---|---|---|
| 13 | *Moraxella* sp. | isolate |
| 14 | *Nitrobacter winogradskyi* | IFO 14297 |
| 15 | *Nitrosomonas europaea* | IFO 14298 |
| 16 | *Paracoccus denitrificans* | IFO 14907 |
| 17 | *Pseudomonas chlororaphis* | IFO 3904T |
| 18 | *Pseudomonas fluorescens* | IFO 14160T |
| 19 | *Pseudomonas pickettii* | JCM 5969T |
| 20 | *Pseudomonas stutzeri* | IFO 14165T |
| 21 | *Rhodococcus erythropolis* | isolate |
| 22 | *Thiobacillus denitrificans* | JCM 3870 |
| 23 | *Thiobacillus novellus* | IFO 12443 |

The test results are shown in Table 3 below. It is noted from Table 3 that the anti-*Nitrospira* antibody prepared in Example 3 hardly showed cross-reactivity with those strains listed in Table 2. This indicates that it is highly specific to *Nitrospira moscoviensis.* Now, it has been found that the highly specific antiserum is useful for easy detection of bacterium belonging to the genus *Nitrospira* which is found in biological sewage treatment systems, such as streams and activated sludge, as well as in water of rivers, lakes and seashore, and in soil habitats, because it causes agglutination with the bacteria of interest, which serves as a direct indication of detection, and additionally the coloration reaction which correlates to the reaction characteristics of the agglutination, which serves as an indirect indication for detection, and it can be made into an antibody-sensitized latex for detection.

Table 3

Cross-reactivity of anti-*Nitrospira* antibody

| No. | Name of Strain | Relative antibody titer (%) |
|---|---|---|
| 1 | *Agrobacterium radiobacter* | 4.8 |
| 2 | *Agrobacterium tumefaciens* | 5.0 |
| 3 | *Alcaligenes denitrificans* | 3.2 |
| 4 | *Alcaligenes faecalis* | 5.0 |
| 5 | *Bacillus licheniformis* | 3.6 |
| 6 | *Bacillus subtilis* | 2.4 |
| 7 | *Enterobacter* sp. | 0.2 |
| 8 | *Flavobacterium ferrugineum* | 0.1 |
| 9 | *Flavobacterium* sp. | 1.3 |
| 10 | *Flavobacterium* sp. | 0.7 |
| 11 | *Flavobacterium* sp. | 2.8 |
| 12 | *Nocardia* amarae | 0.1 |
| 13 | *Moraxella* sp. | 4.4 |
| 14 | *Nitrobacter winogradskyi* | 0.1 |
| 15 | *Nitrosomonas europaea* | 1.3 |
| 16 | *Paracoccus denitrificans* | 0.1 |
| 17 | *Pseudomonas chlororaphis* | 4.3 |
| 18 | *Pseudomonas fluorescens* | 5.1 |
| 19 | *Pseudomonas pickettii* | 8.2 |

(continued)

Cross-reactivity of anti-*Nitrospira* antibody

| No. | Name of Strain | Relative antibody titer (%) |
|-----|----------------|----------------------------|
| 20 | *Pseudomonas stutzeri* | 4.4 |
| 21 | *Rhodococcus erythropolis* | 0.6 |
| 22 | *Thiobacillus denitrificans* | 2.0 |
| 23 | *Thiobacillus novellus* | 5.2 |
| | *Nitrospira moscoviensis* | 100.0 (antigen) |

**Example 5: Preparation of latex reagent for a bacterium belonging to the genus *Nitrospira***

Several kinds of latex reagents for the bacterium belonging to the genus *Nitrospira* were prepared which differ in the amount of protein with which latex is sensitized and also in the particle diameter of latex, so that they have a high detection sensitivity. The antibody-sensitized latex reagents were prepared as follows according to the method disclosed in the aforesaid patent document, JP-A-8029426.

Anti-*Nitrospira* antibody prepared in Example 3 was diluted with GBS to 50-500 µg/mL. The diluted antibody solution was mixed with a 0.25% (w/v) latex solution containing high-density polystyrene latex particles (specific gravity of 1.5 g/mL, from JSR Corp.) diluted with GBS, in equal volumes. The mixture was allowed for reaction at 37°C for 1.0 hour. Then it was given a blocking solution (0.1M GBS containing 1.0% BSA), so that BSA blocked those parts on latex particles which had not adsorbed the antibody.

After blocking for 30 minutes, the latex was centrifuged at room temperature at 2,700 × g for 10 minutes, to remove unbound antibody. The separated latex particles with antibody adsorbed thereon were centrifugally washed twice with a preserving solution (0.1M GBS containing 1.0% BSA and 0.08% NaN$_3$, pH 8.2). They were suspended in a preserving solution of the same composition as above so that the final solution contained 0.05% (w/v) particles. The thus obtained solution was used as the latex reagent for the bacterium belonging to the genus *Nitrospira.* The foregoing procedure was repeated for latex particles differing in grain size as shown in Table 4 below.

Table 4

Diameter of latex particles

| Product No. | Diameter (µm) |
|-------------|---------------|
| H0901 | 0.94 |
| H0902 | 0.98 |
| H1002R | 1.04 |
| H1011 | 1.29 |
| H1009 | 1.35 |
| H1008 | 1.48 |
| H2007R | 2.05 |
| H2001 | 2.26 |
| HS1501R | 2.88 |
| HS1501 | 3.50 |

**Example 6: Detection of a bacterium belonging to the genus *Nitrospira* by reverse passive latex agglutination**

Formalin-fixed *Nitrospira moscoviensis,* strain DSM 10035, with a known cell count, was diluted serially 2-fold with a preserving solution. The resulting diluted solution was dispensed (50 µL each) into 96 wells of microwell UV plate (JS103-20, from Sanko Junyaku Co., Ltd.). Each well was given an equal amount of the high-density latex reagent sensitized with antibody in various concentrations (different degrees of dilution). After thorough stirring by mild vibration without splash, the mixture was allowed for reaction at room temperature for 4.0 hours. The result of reaction indicates the minimum cell count that can be detected. The foregoing test was carried out for each latex sample shown in Table 4 to investigate the relation between the diameter of latex particles most sensitive to detection and the concentration of the sensitizing antibody protein.

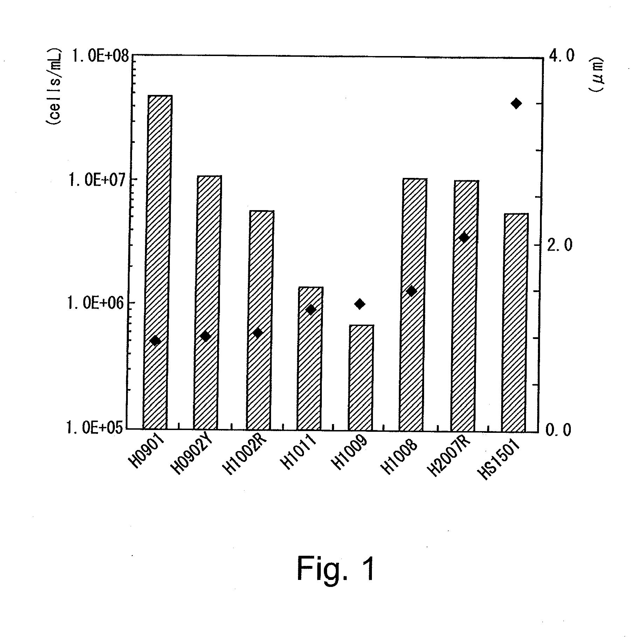

As shown in Fig. 1, the results of the tests indicate that the reagent becomes sensitive in proportion to the diameter within the range of 0.9 to 1.4 $\mu$m. By contrast, sensitivity decreases as the diameter exceeds 1.4 $\mu$m. Moreover, latex particles of the same diameter vary in detection sensitivity depending on the amount of antibody for sensitization. Therefore, it is possible to prepare the latex reagent for the bacterium belonging to the genus *Nitrospira,* which has any desired detection sensitivity, if a proper control is imposed on the diameter of latex particles to be sensitized with an antibody and the amount of antibody protein for sensitization.

It is remarkable to note from Fig. 1 that the latex reagent prepared from Product No. H1009 (high-density latex particles) has the maximum detection sensitivity of $7.0 \times 10^5$ cells/mL. This suggests that the latex reagent for the bacterium belonging to the genus *Nitrospira* according to the present invention has a practically high level of sensitivity for detection and determination of the bacterium belonging to the genus *Nitrospira* which is found in biological sewage treatment systems, such as streams and activated sludge, as well as in water of rivers, lakes and seashore, and in soil habitats, in view of the fact that the cell count of the bacterium belonging to the genus *Nitrospira* ranges from $10^6$ to $10^8$ cells/mL in activated sludge collected from many sewage treatment facilities throughout Japan. This cell count is based on the present inventors' investigation by fluorescence *in situ* hybridization (FISH) method.

**Example 7: Determination of the cell count of bacterium belonging to the genus *Nitrospira* in samples**

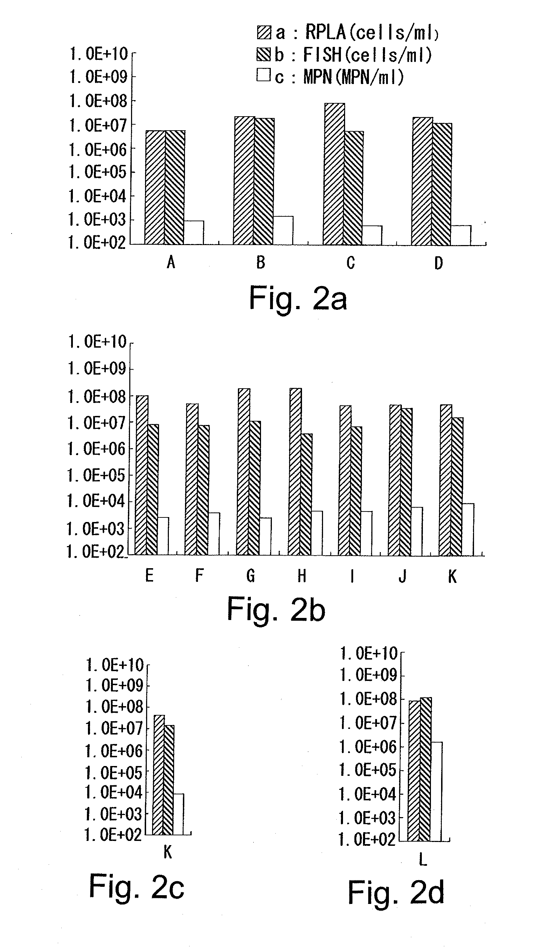

The effectiveness of the immunoassay according to the present invention was confirmed by applying the reverse passive latex agglutination (RPLA) method to detection and determination of a bacterium belonging to the genus *Nitrospira* which is found in activated sludge. Samples of activated sludge were collected from four facilities using the standard activated sludge process (Fig. 2a), six facilities using the oxidation ditch process (Fig. 2b), one facility using the A20 process (Fig. 2c), and one facility using the membrane separation process (Fig. 2d). These 12 facilities are identified by codes A to L in Figs. 2a to 2d.

Samples of activated sludge collected from several facilities were examined for cell count of a bacterium belonging to the genus *Nitrospira* by not only RPLA method but also FISH method for comparison. The FISH method employs DNA probe Ntspa 0662, which is specific to a bacterium belonging to the genus *Nitrospira,* as proposed by Amann et al. (See Amann R. "In situ identification of micro-organisms by whole cell hybridization with rRNA-targeted nucleic acid probes", Molecular Microbial Ecology Manual 3.3.6 (1995) pp. 1-15.) The samples were also examined for cell count of the nitrite oxidizing bacteria by the most probable number (MPN) method which is based on cultivation.

The results are shown in Figs. 2a to 2d. The tests by the RPLA method detected a bacterium belonging to the genus *Nitrospira* in all the samples, and the cell count of the detected bacterium was of the order of $10^6$ to $10^8$ cells/mL. The tests by the FISH method also detected the bacterium in 12 samples, and the cell count of the detected bacterium coincided with that obtained by the RPLA method in 6 samples. In other samples, the measurement by the FISH method is one order of magnitude lower than that by the RPLA method. Incidentally, the results do not vary depending on the method of sewage treatment process.

By contrast, the MPN method gave measurements which are 1-5 orders of magnitude lower than those obtained by the RPLA method. It is known that the MPN method gives a cell count which is 2-5 orders of magnitude lower than that obtained by the antigen-antibody method when applied to nitrifying bacteria in the samples of mixed culture. (See Araki N., Ohashi A., Harada H., and Izarul M., "Cell count of nitrifying bacteria in the biofilm by the FISH method and observation of their special distribution", Journal of Water and Environmental Technology, vol. 22, No. 2, Japan Society of Water Environment (1999) pp. 152-159). The same result as above was obtained by the experiments carried out in this embodiment. Thus, it is apparent that the RPLA method is more suitable than the MPN method for determination of nitrifying bacteria.

The FISH detection method relies on the fact the base sequence of 16S-ribosomal DNA and RNA varies from one bacterium to another, and hence it is considered to be very useful for molecular biological analysis of environmental microorganisms. However, it is based on the assumption that there exist many targets (ribosomal RNA) in cells and hence it cannot be applied to cells without sufficient ribosomal RNA (such as starving cells). On the other hand, those microorganisms living in activated sludge used for the biological nitrogen treating process usually do not have sufficient ribosomal RNA because they are subject to environmental stress due to diversity of influent water. Therefore, it is assumed in this experiment that there exist the bacterium belonging to the genus *Nitrospira* which has sufficient nitrite oxidizing activity but is not fully detected by the FISH method.

The RPLA method, which employs an antibody, would count dead cells which have lost activity but still possess residual antigenicity. This is a probable reason why the cell count of the bacterium belonging to the genus *Nitrospira* measured by the RPLA method is higher than that measured by the FISH method.

Nevertheless, it was confirmed that the RPLA method which employs the antibody-sensitized latex particles according to the present invention excels in rapidity and ease with which it is used, so long as it is intended to rapidly identify and determine a bacterium belonging to the genus *Nitrospira* which is found in biological sewage treatment systems, such as streams and activated sludge, as well as in water of rivers, lakes and seashore, and in soil habitats.

**Example 8: Application to operation management of sewage treatment facilities**

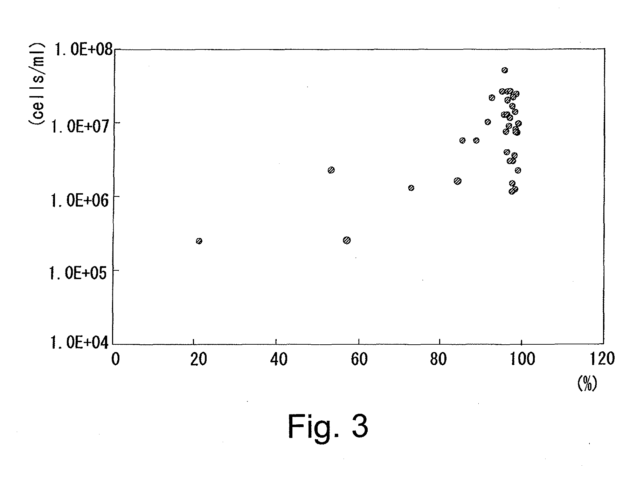

Experiments were carried out to see the relation between the cell count of the bacterium belonging to the genus *Nitrospira* which is found in the nitrifying tank and the amount of Kjeldahl nitrogen in influent and treated water. Samples were collected from 10 facilities at every season (spring, summer, autumn and winter), and hence 40 samples in total were analyzed. The results are shown in Fig. 3. It is noted from Fig. 3 that more than 90% of Kjeldahl nitrogen is removed by treatment in the reactors in which the cell count of bacteria of the genus *Nitrospira* is greater than $10^7$ cells/mL. It is also apparent that more than 80% of Kjeldahl nitrogen is removed by treatment in about 90% of the reactors in which the cell count is $10^6$ to $10^7$ cells/mL.
The ratio of nitrification is expressed by the following formula.

$$\text{Ratio of nitrification (\%)} = \frac{A-B}{A} \times 100$$

where,

A: Concentration of Kjeldahl nitrogen in influent water
B: Concentration of Kjeldahl nitrogen in treated water

The foregoing suggests that a ratio of nitrification higher than 80% can be maintained if the nitrifying reactor is run in such a way that the cell count of the bacterium belonging to the genus *Nitrospira* therein is greater than $10^6$ cells/mL, preferably greater than $10^7$ cells/mL.
Since the growth rate of nitrifying bacteria is much lower than that of bacteria that decompose organic matter, the condition expressed by the formula (1) below, which permits nitrifying bacteria to live in the system, should be satisfied if the same reactor is to be used for oxidative decomposition of C-BOD and nitrification simultaneously like the biological nitrification-denitrification process.

$$\text{SRTN} \geq 1/\mu\text{N} \qquad\qquad \dots \ (1)$$

where,

SRTN: residence time (days) of solids to hold nitrifying bacteria in the reactor

$\mu$N: specific growth rate of nitrifying bacteria

In the sewage treatment process with activated sludge, the SRT is the same as that of nitrifying bacteria. Consequently, for the treatment process to accelerate nitrification while keeping nitrifying bacteria, it is necessary to establish the SRT that satisfies the formula (1).
Since the growth rate of nitrifying bacteria is affected by the water temperature, it is necessary to extend the solids residence time (SRT) necessary for nitrifying bacteria to stay in the reactor at the period of low water temperature. Sewage treatment facilities designed for the standard activated sludge process have conventionally been operated according to the SRT for each water temperature enough for complete nitrification which is empirically obtained from the relation between the SRT and the temperature for nitrification. This method of operation control is based entirely on the empirical formula and the SRT derived from it does not necessarily lead to the maximum ratio of nitrification in all reactors.
By contrast, the antibody-sensitized latex of the present invention allows for rapid and easy determination of nitrifying bacteria in the reactor and the resulting data permits the SRT to be adjusted so that the reactor keeps as many bacteria as necessary for the adequate ratio of nitrification. Thus, the reactors can be controlled individually. Incidentally, although the MPN method, FISH method, and quantitative PCR method may be used to determine nitrifying bacteria, they are not suitable for handy use in ordinary purification facilities because they need long time, skillful work and expensive equipment and reagents.

Controlling the sewage treatment process according to an accurate cell count has become a reality with the advent of the present invention. Apparently, this new controlling method is more effective than the conventional ones. In summary, the present invention provides the antibody-sensitized latex for determination which comprises latex particles and an antibody adsorbed thereon, the antibody being specific to a bacterium belonging to the genus *Nitrospira*. The antibody-sensitized latex allows for rapid and easy detection and determination of the bacterium belonging to the genus *Nitrospira* unlike the conventional technology which needs special skillful work and long time for determination.

Through reactions with an antibody specific to the bacterium belonging to the genus *Nitrospira,* the antibody-sensitized latex according to the present invention allows for rapid and easy cell count of the bacterium belonging to the genus *Nitrospira* which is found in biological sewage treatment systems, such as streams and activated sludge, as well as in water of rivers, lakes and seashore, and in soil habitats.

An additional advantage of the present invention is that the cell count determined as mentioned above can be used as an index to control sewage treatment facilities in an easy way.

**Brief Description of the Drawings**

Fig. 1 is a bar graph showing the relation between the diameter of latex particles and the lower limit of measurement, in which the left ordinate represents the lower limit of measurement (cells/mL), the right ordinate represents the latex diameter ($\mu$m) marked with ♦, and the abscissa represents the product number of latex particles.

Figs. 2a to 2d are bar graphs, each showing the cell count of a bacterium belonging to the genus *Nitrospira* measured by the RPLA method, FISH method, and MPN method, in which the abscissas in Figs. 2a to 2d represent respectively purification facilities A to D by standard activated sludge method, purification facilities E to J by oxidation ditch method, purification facilities K by A20 method, and purification facilities L by membrane separation method, from which samples of activated sludge were collected, the ordinates represent the cell count (cells/mL) for the RPLA method and FISH method and the cell count (MPN/mL) for the MPN method, and the bars a, b and c represent respectively the results measured by the RPLA method, FISH method, and MPN method.

Fig. 3 is a diagraph showing the relation between the cell count of a bacterium belonging to the genus *Nitrospira* and the ratio of nitrification, in which the ordinate represents the cell count (cells/mL), the abscissa represents the ratio (%) of nitrification (removal of Kjeldahl nitrogen from water for treatment), and plots in the diagram represent 40 samples used for measurement.

**Claims**

1. An antibody-sensitized latex for detecting a bacterium belonging to the genus *Nitrospira,* comprising a liquid medium and latex particles suspended therein, wherein each of said latex particles has a specific gravity of about 1. 5 g/mL and an average particle diameter in the range of 1.0-1.5 $\mu$m, and wherein each of said latex particles carries an antibody adsorbed thereon, said antibody being specific to a bacterium belonging to the genus *Nitrospira* having an activity of nitrite oxidization.

2. A method for detecting a bacterium belonging to the genus *Nitrospira* by means of the antibody-sensitized latex as defined in Claim 1, said method comprising the steps of:

   mixing said antibody-sensitized latex with a test sample to obtain a mixture;
   allowing the mixture to stand for a predetermined period of time; and
   observing the presence or absence of agglutination of latex particles in the mixture.

3. The method according to Claim 2, wherein the test sample is one which is collected from liquid being treated in a vessel for sewage treatment with aerobic bacteria.

4. A method for determining the cell count of a bacterium belonging to the genus *Nitrospira* by means of the antibody-sensitized latex as defined in Claim 1, said method comprising the steps of:

   preparing a plurality of test samples which are different in degree of dilution of a sample to be determined;
   mixing each of the test samples with said antibody-sensitized latex to obtain a plurality of mixtures;
   allowing the mixtures to stand for a predetermined period of time;
   observing the presence or absence of agglutination of latex particles in each of said mixtures;
   comparing the result of observation of the mixtures with agglutination with the result of a reference test in which a procedure according to the foregoing steps are followed for a plurality of reference samples each of which is assigned to a known cell count of the bacterium belonging to the genus *Nitrospira;* and

identifying the cell count of the bacterium belonging to the genus *Nitrospira* in the test samples on the basis of comparison of the result of observation of the mixtures with the result of the reference test which correspond to agglutination that observed in the mixtures.

5. The method according to Claim 4, wherein said sample is one which is collected from liquid being treated in a vessel for sewage treatment with aerobic bacteria.

6. The method according to Claim 5, after measuring the cell count of a bacterium belonging to the genus *Nitrospira,* further comprising the step of:

controlling at least one condition for operation selected from the group consisting of the residence time of solids in the reactor, the amount of dissolved oxygen in the reactor, and the inflow into the reactor, based on the measurement of the cell count of the bacterium, so as to maintain the cell count of the bacterium at a cell count value necessary for the liquid in the reactor to keep the intended ratio of nitrification during treatment.

7. The method according to Claim 6, wherein the cell count of the bacterium belonging to the genus *Nitrospira,* which is necessary for the liquid in the reactor to keep the intended ratio of nitrification, is maintained at $10^7$ cells/mL or more.

Fig. 1

a : RPLA(cells/ml)
b : FISH(cells/ml)
c : MPN(MPN/ml)

Fig. 2a

Fig. 2b

Fig. 2c

Fig. 2d

Fig. 3

## INTERNATIONAL SEARCH REPORT

| | International application No. |
|---|---|
| | PCT/JP2005/013807 |

**A. CLASSIFICATION OF SUBJECT MATTER**
*G01N33/545*(2006.01)

According to International Patent Classification (IPC) or to both national classification and IPC

**B. FIELDS SEARCHED**

Minimum documentation searched (classification system followed by classification symbols)
*G01N33/545*(2006.01)

Documentation searched other than minimum documentation to the extent that such documents are included in the fields searched
Jitsuyo Shinan Koho        1922-1996   Jitsuyo Shinan Toroku Koho   1996-2005
Kokai Jitsuyo Shinan Koho   1971-2005   Toroku Jitsuyo Shinan Koho   1994-2005

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 8-29426 A  (Yakult Honsha Co., Ltd.), 02 February, 1996 (02.02.96), & EP 752586 A          & WO 1995/25958 A | 1-7 |
| A | JP 5-322896 A  (Yakult Honsha Co., Ltd.), 07 December, 1993 (07.12.93), (Family: none) | 1-7 |
| A | JP 2002-27976 A  (Sumitomo Chemical Co., Ltd.), 29 January, 2002 (29.01.02), (Family: none) | 1-7 |

☐ Further documents are listed in the continuation of Box C.        ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered   to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 24 October, 2005 (24.10.05) | 08 November, 2005 (08.11.05) |

| Name and mailing address of the ISA/ | Authorized officer |
|---|---|
| Japanese Patent Office | |
| Facsimile No. | Telephone No. |

Form PCT/ISA/210 (second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5322896 A

- JP 8029426 A

**Non-patent literature cited in the description**

- Methods for Experiments on Soil Microorganisms. **RYUSUKE KIMURA et al.** Society for the Research of Soil Microorganisms. Yokendo Co., Ltd, 1992, 207-214
- **EHRICH S. ; D. BEHRENS ; E. LEBEDEVA ; W. LUDWIG ; E. BOCK.** A new obligately chemolitoautotrophic, nitrite-oxidizing bacterium, Nitrospira moscoviensis sp. nov. and its phylogenetic relationship. *Arch. microbiol.,* 1995, vol. 164, 16-23

- In situ identification of micro-organisms by whole cell hybridization with rRNA-targeted nucleic acid probes. **AMANN R.** Molecular Microbial Ecology Manual. 1995, vol. 3.3.6, 1-15
- **ARAKI N. ; OHASHI A. ; HARADA H. ; IZARUL M.** Cell count of nitrifying bacteria in the biofilm by the FISH method and observation of their special distribution. *Journal of Water and Environmental Technology,* 1999, vol. 22 (2), 152-159